# EUROPEAN PATENT SPECIFICATION

(11) **EP 2 089 688 B1**
(45) Date of publication and mention of the grant of the patent: **25.05.2011**
(21) Application number: 07835427.1
(22) Date of filing: 13.11.2007
(51) Int. Cl.: G01N 21/15, A01J 5/013, A01J 7/02, G01N 33/04, G01N 15/14, G01N 21/05

(54) **A DEVICE AND A METHOD FOR SAMPLING OF MILK**
VORRICHTUNG UND VERFAHREN ZUR ENTNAHME VON MILCHPROBEN
DISPOSITIF ET PROCÉDÉ D'ÉCHANTILLONNAGE DE LAIT

(30) Priority: 16.11.2006 SE 0602439
(43) Date of publication of application: 19.08.2009
(73) Proprietor: DeLaval Holding AB, 147 21 Tumba (SE)
(72) Inventor: AXELSON, Johan, 168 53 Bromma (SE); GUDMUNDSSON, Mats, 152 57 Södertälje (SE); FLENNERT, Thomas, 141 70 Segeltorp (SE); SELANDER, Olle, 141 71 Segeltorp (SE)
(74) Representative: Keijser Bergöö, Malin Katarina
(86) International application number: PCT/SE2007/050844
(87) International publication number: WO 2008/060235

(56) References cited:
- EP-A1- 0 252 762
- EP-A1- 1 000 535
- WO-A1-91/13340
- WO-A1-92/19954
- WO-A1-96/16536
- WO-A1-03/090522
- WO-A1-2004/073691
- WO-A1-2005/027624
- GB-A- 1 497 698
- JP-A- 61 052 273
- US-A- 4 647 540
- US-A1- 2005 123 174
- US-A1- 2006 249 082

## Description

### THE BACKGROUND OF THE INVENTION AND PRIOR ART

The present invention relates to a device and a method for sampling of milk, wherein the device comprises a measuring chamber arranged to receive milk samples from animals, a camera adapted to record images of milk samples in the measuring member and image analysing means adapted to count somatic cells and/or fat droplets in the images.

Somatic cell count (SCC) defines the number of white cells per millilitre of milk. SCC scores are used as an international standard in determining the quality and the price of the milk. In order to determine the number of somatic cells in a milk sample, it is extremely important that the milk sample does not contain milk residuals from the previously milked cow, so-called carry over. In such a case, the measurement result could be completely incorrect. In an automatic milk arrangement, it is a desired to provide an automatic analysis of the number of somatic cell in the milk from individual cows. In such a case, a quick and effective washing has to be provided of all components and all surfaces of the sampling device which come in contact with the milk.

WO 03090522 shows a device for sampling of milk. In this case, milk is used to clean the passage through which milk samples are delivered from a milk line to an analysing device. The cleaning of components and surfaces inside the analysing device is performed by water.

### SUMMARY OF THE INVENTION

The object of the present invention is to provide a device and a method which guarantee that components and surfaces inside an analysing device are completely cleaned from milk residues from a previously milk sample when a new milk sample has to be taken.

This object is achieved by the device defined in claim 1 . The already existing camera and image analysing means are here used to supervise if the measuring chamber has been completely cleaned from milk residues before a new milk sample has to be taken. Such a supervision of the cleaning process is very simple to perform, it is reliable and it does not require any additional components.

According to a preferred embodiment of the invention, said cleaning means is adapted to provide a flow of the cleaning liquid through the device along a path such that it comes in contact with the same components and the same surfaces as the milk comes in contact with into the device. Thereby, all components and surfaces inside the device, which have been in contact with milk, will be effectively cleaned by means of the cleaning liquid flow. The cleaning liquid is adapted to provide a flow in contact with a delivery funnel which primary is adapted to collect milk to be tested. Thereby, the delivery funnel may be cleaned from milk residues in an effective manner. The cleaning liquid may be adapted to be transported by means of a pump which primary is used to transport the milk to the measuring chamber. Consequently, any additional pump or the like has not to be used which provides the flow of cleaning liquid through the device. The cleaning liquid may be adapted to be guided to the measuring chamber via a conduit which primary is used to guide the milk to the measuring chamber. Thereby, the inner surfaces of the conduit will be cleaned from milk residues in a simple manner.

According to another preferred embodiment of the invention, said cleaning liquid is water. Water is an effective cleaning liquid and it does not contain any chemical substances which can influence on the result of a milk sample. However, it is possible to use water with suitable detergents In order to further increase the cleaning effect of the cleaning process.

According to another preferred embodiment of the invention, the result of the counting of the somatic cells and/or fat droplets in the image of the cleaned measuring chamber may be disclosed on a screen, Thereby, an operator will notice if the device has been completely cleaned such that a new milk sample can be analysed. The cleaning means is adapted to provide a further flow of a cleaning liquid through the measuring chamber if any somatic cells and/or fat droplets are detected in the image of the cleaned measuring chamber. Such a further cleaning process is started automatically if the device has not been completely cleaned from milk residues from a previous milk sample.

According to another preferred embodiment of the invention, the device comprises means for supplying a staining solution to the milk sample in order to facilitate the counting of somatic cells or fat droplets in the images. The somatic cells change in colour by means of the staining solution such that they are more easy to detect in an image and thus more easy to count. The device may constitute a component in a milking robot. The device is especially suitable to use in an automatically controlled milking arrangement comprising a milking robot.

The above-mentioned object is also achieved by the method defined in claim 6 . In such a manner, a simple and an effective supervision of a cleaning process in an analysing device is obtained.

### BRIEF DESCRIPTION OF THE DRAWINGS

The present invention is now to be explained more closely by means of a preferred embodiment, which is disclosed as an example, and with reference to the attached drawings.
Fig. 1 shows a sampling device for milk with an analysing device and
Fig. 2 shows the analysing device more in detail.

### DETAILED DESCRIPTION OF A PREFERRED EMBODIMENT OF THE INVENTION

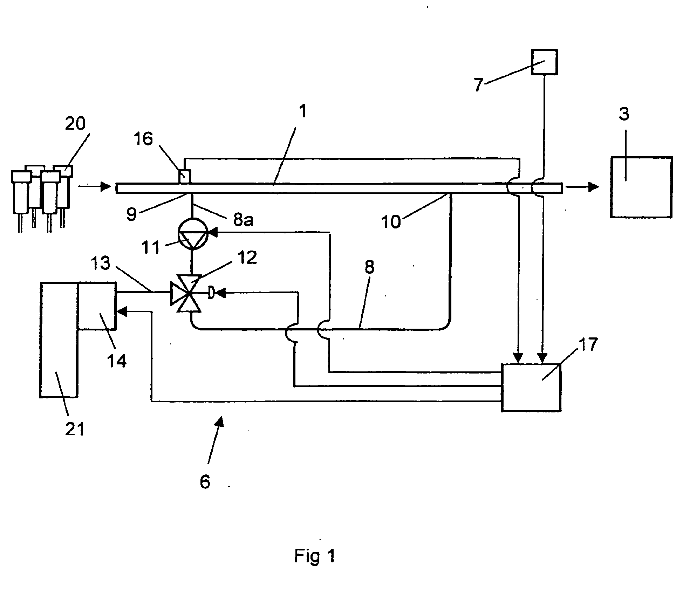

Fig 1 shows a milk line 1 having an extension from a claw 2 to a milk receiver 3. The milk receiver 3 is arranged to store the milk from a cow temporarily. A sampling device 6 is adapted to allow sampling of the milk in the milk line 1. Since the milk line 1 has an extension from only one claw 2, the milk in the milk line 1 originates from one cow at a time. In order to identify the milked cow, a reading device 7 is arranged to read a specific code from a transponder of the cow. The sampling device 6 comprises a conduit loop 8 having an inlet opening at a first end 9, which is connected to a milk line 1, and an outlet opening at a second end 10. Thereby, a part of the milk, which flows in the milk line 1, is allowed to flow into the inlet opening at the first end 9 of the conduit loop 8. The milk in the conduit loop 8 is guided back to the milk line 1, via the outlet opening in the second end 10. Since the second end 10 is connected to the milk line 1 at a distance downstream of the first end 9, the milk, which has passed through the conduit loop 8, is prevented to return to the conduit loop 8. The conduit loop 8 has a substantially smaller inner cross-section area than the milk line 1. Thereby, only a relatively small amount of the milk, which flows in the milk line 1, is sucked in the conduit loop 8.

A pump 11 and a three-way valve 12 are arranged in the conduit loop 8. The pump 11 is positioned in a first part 8a of the conduit loop 8, which first part 8a has an extension from the first end 9 to the three-way valve 12. The three-way valve 12 is mostly positioned in a first position, in which it allows the milk to pass through the valve 12 in a direction towards the outlet opening 10. When a control unit 17 initiates that a milk sample is to be taken, the three-way valve 12 is switched to a second position. In the second position the three-way valve 12 discharges the milk flow from the first part of conduit loop 8a to a second conduit 13, which guides the milk to a schematically disclosed analysing device 14. The analysing device 14 is adapted to count somatic cells or fat droplets in the milk samples. The analysing device 14 may be an integrated part of a schematically disclosed milking robot 21. Consequently, the milk to be sampled is transported through a passage, which has an extension from the milk line 1 to the device 14. This passage comprises the first part 8a of the conduit loop 8 and the second conduit 13. A flow meter 16 is arranged in the milk line 1 in order to measure the flow rate in the milk line 1. The control unit 17 is arranged to supervise and control the milk sampling process. By using a control unit 17, a substantially automatic sampling of the milk from individual cows is possible to accomplish.

When a cow has entered, for example, a milking stall, the milking robot 21 attaches the teat cups 20 to the cow. The reading device 7 reads the identity of the cow and informs the control unit 17 about the identity of the cow by a signal. The milking process starts and the milk from the cow begins to flow in the milk line 1. The milk in the milk line 1 is transported in a direction towards a milk receiver 3. The flow meter 16 detects the milk flow in the milk line 1. The flow meter 16 informs the control unit 17 by a signal about the presence of the flow and the flow value in the milk line 1. The control unit 17 initiates activation of the pump 11, which establishes a negative pressure at the first end 9 of the conduit loop 8. Thereby, a part of the milk, which flows in the milk line 1, is sucked in the conduit loop 8, via the inlet opening, at the first end 9.

The milk flow, from the cow, removes possible milk residues in the conduit loop 8 from the previously milked cow. Such milk residues may be stored as a thin film along the inner wall surface of the conduit loop 8. The milk from the cow, which is milked, provides a very simple, quick and effective rinsing of the conduit loop 8 from milk residues. Thereby, remaining milk residues in the first part 8a of the conduit loop 8 will not influence on a milk sample of the presently milked cow. The control unit 17 initiates sampling of the milk from the cow only after that a time period has passed and/or a suitable amount of milk from the cow has flown through the three-way valve 12 in the conduit loop 8. When, such a suitable amount of milk has flown through the three-way valve 12, the risk that milk residues from the previously milked cow remains in the first part 8a of the conduit loop 8 is substantially eliminated.

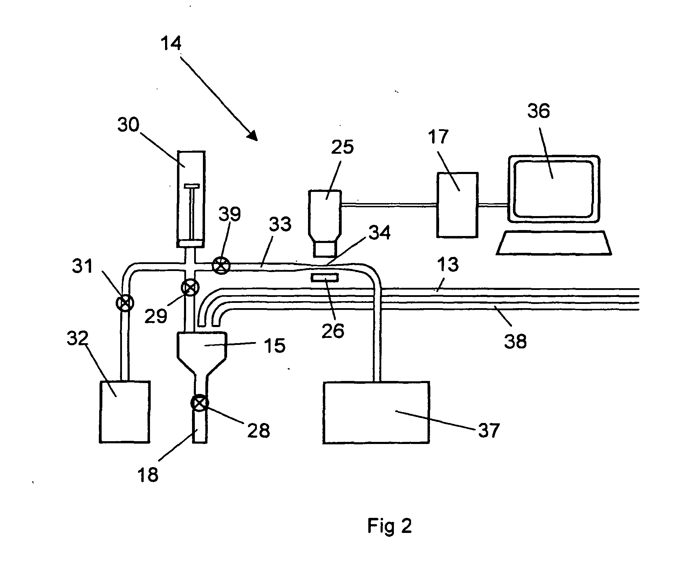

When a sample of the milk from the cow is to be taken, the control unit 17 initiates an adjustment of the three-way valve 12 from the first position to the second position. The milk in the first part 8a of the conduit loop 8 is now directed into the second conduit 13 and the analysing device 14. The analysing device 14 is shown in more detail in Fig. 2. The milk from the second conduit is enters a delivery funnel 15. The delivery funnel 15 comprises a valve 28. Initially, the valve 28 is in an open state such that the milk in the funnel 15 is drained out through an outlet conduit 18. The outlet conduit 18 may transport this milk back to the conduit loop 8 downstream of the three-way valve 12 or to a specific container for milk to be discarded. Thereby, the initial milk flow through the second conduit 13 removes milk residues in the second conduit 13 from a previously tested cow. Consequently, the milk rinses the first part 8a of the conduit loop and the second conduit 13 from milk residues.

When a determined amount of milk has passed the delivery funnel 15, the valve 28 is closed. The milk flow in the second conduit 13 fills now the delivery funnel 15. When the delivery funnel 15 is filled with milk, a valve 29 is open. A syringe pump 30 is activated such that a quantity of milk is sucked from the delivery funnel 15 into an inner space of the syringe pump 30. The valve 29 is closed and a valve 31 is open. During a further activation of the syringe pump, a staining solution is sucked from a container 32 into the inner space of the syringe pump 30. The milk and the staining solution is mixed in the syringe pump 30. The mixture of milk and the staining solution changes in colour which makes it possible to more clearly indicate the somatic cells in the milk sample. The valve 31 is closed and a valve 39 is open. The syringe pump 30 discharges the mixture of the milk and the staining solution into a conduit 33. The conduit 33 comprises a narrow portion in the form of a measuring chamber 34 with transparent walls. A camera 25 is arranged at one wall surface of the measuring chamber 34 and a light source 26 is arranged at the opposite wall of the measuring chamber 34. The camera records an image of milk sample in the collecting member 34. The camera 25 may comprise a microscope such that images of the stagnant milk in a small area of the measuring chamber 34 may be recorded. In this area, the measuring chamber 34 may have a thickness of about 0,1 mm. The control unit 17 comprises image analysing means. The camera 25 sends images to the control unit 17 and the image analysing means counts the somatic cells and/or fat droplets in the images. By recording a large number of images and by means of digital processing of these images, a somatic cell count score can be determined. The result is disclosed on a screen 36. When the milk sample has passed the measuring chamber 34 it is drained to a waste container 37. The valve 28 is open such that the remaining milk in the delivery funnel 15 is drained out through the outlet conduit 18. The three-way valve 12 is switched back to the first position. The control unit 17 stores the received information about the quality of the milk from the cow. The milk may be stored in the milk receiver 3 until the control unit 17 has received information about the quality of the milk from the device 14. If the milk from said cow does not perform determined requirements of the quality, the milk in the milk receiver 3 is discarded.

The device comprises cleaning means adapted to clean the measuring chamber 34 and all other spaces in the device 14 between each milk sampling process. The cleaning means supplies a cleaning liquid to the delivery funnel 15 by means of a conduit 38. Preferably, the cleaning liquid is water. The valve 28 is closed such that cleaning liquid will fill the delivery funnel 15. The valve 29 is open and the syringe pump 30 is activated such that cleaning water is sucked from the delivery funnel 15 into the syringe pump 30. The valve 29 is closed and the syringe pump 29 discharges the cleaning liquid through the conduit 33 and the measuring chamber 34 to the waste container 37. After that, the camera records at least one image of the measuring chamber 34. The image is sent the image analysing means of the control unit 17, which is adapted to count the somatic cells and/or fat droplets of the image of the cleaned measuring chamber 34. The result is disclosed on the screen 36. If no somatic cells and/or fat droplets are detected, the cleaning process has been successful and the analysing device is ready to perform a new analyse of a new milk sample. If somatic cells and/or fat droplets are detected, the cleaning process has not been successful and the cleaning process will be repeated. However, in certain cases, a low number of somatic cells can be accepted. A threshold valve can define an acceptable number of somatic cells.

The invention is not restricted to the described embodiments of the invention but may be varied freely within the scope of the claims. The above-described analysing device may have an arbitrary location, can also be placed between the milk receiver 3 and 9 milk tank. It is not restricted to be used with a sample device of the kind disclosed in Fig. 1.

## Claims

1. A device for sampling of milk, wherein the device (14) comprises a measuring chamber (34) arranged to receive milk samples from animals, a camera (25) adapted to record images of the milk samples in the measuring chamber (34) and image analysing means adapted to count somatic cells and/or fat droplets in the images, **characterised in that** the device (14) comprises cleaning means adapted to provide a flow of a cleaning liquid through the measuring chamber (34) after a milk sample has been taken, that the camera (25) is adapted to record at least one image of the measuring chamber (34) after it has been cleaned by the cleaning liquid, that the image analysing means (17) is adapted to count the somatic cells and/or fat droplets in the image of the cleaned measuring chamber (34) and that the cleaning means is adapted to provide a further flow of a cleaning liquid through the measuring chamber (34) if any somatic cells and/or fat droplets are detected in the image of the cleaned measuring chamber (34), wherein such a further cleaning process is started automatically if the device has not been completely cleaned from milk residues from a previous milk sample.

2. A device according to claim 1, **characterised in that** said cleaning means is adapted to provide a flow of the cleaning liquid through the device (14) along a path such that it comes in contact with the same components (15, 30, 33, 34) and the same surfaces as the milk comes in contact with into the device (14).

3. A device according to claim 1 or 2, **characterised in that** the cleaning liquid is adapted to provide a flow in contact with a delivery funnel (15) which primarily is adapted to collect milk to be tested.

4. A device according to any one of the preceding claims, **characterised in that** the device (14) comprises means for supplying a staining solution to the milk sample in order to facilitate the counting of somatic cells or fat droplets in the images.

5. A device according to any one of the preceding claims, **characterised in that** the device constitutes a component in a milking robot (21).

6. A method for sampling of milk, wherein the method comprises the steps of receiving milk samples from animals in a measuring chamber (34), recording images of milk samples in the measuring chamber (34), and counting somatic cells and/or fat droplets in the images, providing a flow of a cleaning liquid through the measuring chamber (34) after a milk sample has been taken, recording at least one image of the measuring chamber (34) after it has been cleaned by the cleaning liquid, counting the somatic cells and/or fat droplets in the image of the cleaned measuring chamber (34) and providing a further flow of a cleaning liquid through the measuring chamber (34) if any somatic cells and/or fat droplets are detected in the cleaned measuring chamber (34).

7. A method according to claim 6, **characterised by** the step of providing a flow of the cleaning liquid through the device (14) along a path such that it comes in contact with the same components (15, 30, 33, 34) and the same surfaces as the milk comes In contact with into the device (14).

8. A method according to claim 6 or 7, **characterised by** the step of providing a flow of the cleaning liquid in contact with a delivery funnel (15) which primarily is adapted to collect milk to be tested.

9. A method according to any one of the preceding claims 6 to 8, **characterised by** the step of transporting the cleaning liquid by means of a pump (30) which primarily is used to transport the milk to the measuring chamber (34).

10. A method according to any one of the preceding claims 6 to 9, **characterised by** the step of guiding the cleaning liquid to the measuring chamber (34) in a conduit (33) which primarily is used to guide the milk to the measuring chamber (34).

11. A method according to any one of the preceding claims 6 to 10, **characterised by** the step of using water as cleaning liquid.

12. A method according to any one of the preceding claims 6 to 11, **characterised by** the step of disclosing the result of the counting of the somatic cells and/or fat droplets in the image of the cleaned measuring chamber (34) on a screen (36).

13. A method according to any one of the preceding claims 6 to 13, **characterised by** the step of supplying a staining solution to the milk sample in order to facilitate the counting of somatic cells or fat droplets in the images.

## Patentansprüche

1. Vorrichtung zur Entnahme von Milchproben, wobei die Vorrichtung (14) eine Messkammer (34), die angeordnet ist, Milchproben von Tieren zu empfanden, eine Kamera (25), die angepasst ist, Bilder der Milchproben im Messkammer (34) aufzuzeichnen und ein Bildanalysemittel, das angepasst ist, somatische Zellen und/oder Fetttropfen in den Bildern zu zählen, umfasst, **dadurch gekennzeichnet, dass** die Vorrichtung (14) ein Reinigungsmittel umfasst, das angepasst ist, einen Fluss einer Reinigungsflüssigkeit durch die Messkammer (34) bereitzustellen, nachdem eine Milchprobe genommen worden ist, dass die Kamera (25) angepasst ist, mindestens ein Bild der Messkammer (34) aufzuzeichnen, nachdem sie von der Reinigungsflüssigkeit gereinigt worden ist, dass das Bitdanatysemittel (17) angepasst ist, die somatischen Zellen und/oder Fetttropfen in dem Bild der gereinigten Messkammer (34) zu zählen, und dass das Reinigungsmittel angepasst ist, einen weiteren Fluss einer Reinigungsflüssigkeit durch die Messkammer (34) bereitzustellen, wenn irgendwelche somatischen Zellen und/oder Fetttropfen in dem Bild der gereinigten Messkammer (34) erfasst worden sind, wobei solch ein weiterer Reinigungsprozess automatisch gestartet wird, wenn die Vorrichtung nicht vollständig von Milchrückständen von einer vorhergehenden Milchprobe gereinigt worden ist.

2. Vorrichtung nach Anspruch 1, **dadurch gekennzeichnet, dass** das Reinigungsmittel angepasst ist, einen Fluss der Reinigungsflüssigkeit durch die Vorrichtung (14) entlang einem Pfad bereitzustellen, so dass sie in Kontakt mit den gleichen Komponenten (15, 30, 33, 34) und den gleichen Flächen kommt, mit denen die Milch in der Vorrichtung (14) in Kontakt kommt.

3. Vorrichtung nach Anspruch 1 oder 2, **dadurch gekennzeichnet, dass** die Reinigungsflüssigkeit angepasst ist, einen Fluss bereitzustellen, der in Kontakt mit einem Abgabetrichter (15) steht, der in erster Linie angepasst ist, Milch zu sammeln, die getestet werden soll.

4. Vorrichtung nach einem der vorhergehenden Ansprüche, **dadurch gekennzeichnet, dass** die Vorrichtung (14) ein Mittel zum Zuführen einer Färbungslösung zu der Milchprobe umfasst, um das Zählen der somatischen Zellen oder der Fetttropfen in den Bildern zu erleichtern.

5. Vorrichtung nach einem der vorhergehenden Ansprüche, **dadurch gekennzeichnet, dass** die Vorrichtung eine Komponente in einem Melkroboter (21) darstellt.

6. Verfahren zur Entnahme von Milchproben, wobei das Verfahren die folgenden Schritte umfasst:
Empfangen von Milchproben von Tieren in einer Messkammer (34), Aufzeichnen von Bildern von Milchproben im Messkammer (34) und Zählen somatischer Zellen und/oder Fetttropfen in den Bildern, Bereitstellen eines Flusses einer Reinigungsflüssigkeit durch die Messkammer (34), nachdem eine Milchprobe gekommen worden ist, Aufzeichnen mindestens eines Bilds der Messkammer (34), nachdem sie von der Reinigungsflüssigkeit gereinigt worden ist, Zählen der somatischen Zellen und/oder Fetttropfen in dem Bild der gereinigten Messkammer (34) und Bereitstellen eines weiteren Flusses einer Reinigungsflüssigkeit durch die Messkammer (34), wenn irgendwelche somatischen Zellen und/oder Fetttropfen in der gereinigten Messkamner (34) erfasst werden.

7. Verfahren nach Anspruch 6, **gekennzeichnet durch** den Schritt des Bereitstellens eines Flusses der Reinigungsflüssigkeit **durch** die Vorrichtung (14) entlang einem Pfad, so dass sie in Kontakt mit den gleichen Komponenten (15, 30, 33, 34) und den gleichen Flächen kommt, mit denen die Milch in der Vorrichtung (14) in Kontakt kommt.

8. Verfahren nach Anspruch 6 oder 7, **gekennzeichnet durch** den Schritt des Bereitstellens eines Flusses der Reinigungsflüssigkeit in Kontakt mit einem Abgabetrichter (15), der in erster Linie angepasst ist, Milch zu sammeln, die getestet werden soll.

9. Verfahren nach einem der vorhergehenden Ansprüche 6 bis 8, gekenntzeichnet durch den Schritt des Transportierens der Reinigungsflüssigkeit mittels einer Pumpe (30), die in erster Linie verwendet wird, um die Milch durch die Messkammer (34) zu transportierten.

10. Verfahren nach einem der vorhergehenden Ansprüche 6 bis 9, **gekennzeichnet durch** den Schritt des Leitens der Reinigungsflüssigkeit zur Messkammer (34) in einem Kanal (33), der in erster Linie verwendet wird, um die Milch zur Messkammer (34) zu leiten

11. Verfahren nach einem der vorhergehenden Ansprüche 6 bis 10, **gekennzeichnet durch** den Schritt des Verwendens von Wasser als Reinigungsflüssigkeit.

12. Verfahren nach einem der vorhergehenden Ansprüche 6 bis 11, **gekennzeichnet durch** den Schritt des Offenlegens des Ergebnisses des Zählens der somatischen Zellen und/oder- Fetttropfen in dem Bild der gereinigten Messkammer (34) auf einem Bildschirm (36).

13. Verfahren nach einem der vorhergehenden Ansprüche 6 bis 13, **gekennzeichnet durch** den Schritt des Zuführens einer Färbungslösung zu der Milchprobe, um das Zählen der somatischen Zellen oder Fetttropfen in den Bildern zu erleichtern.

## Revendications

1. Dispositif d'échantillonnage de lait, dans lequel le dispositif (14) comprend une chambre de mesure (34) agencée pour recevoir des échantillons de lait d'animaux, une caméra (25) adaptée pour enregistrer des images des échantillons de lait dans la chambre de mesure (34) et un moyen d'analyse d'image adapté pour compter des cellules somatiques et/ou des gouttelettes de graisse dans les images, **caractérisé en ce que** le dispositif (14) comprend un moyen de nettoyage adapté pour fournir un flux d'un liquide de nettoyage à travers la chambre de mesure (34) après qu'un échantillon de lait a été pris, **en ce que** la caméra (25) est adaptée pour enregistrer au moins une image de la chambre de mesure (34) après qu'elle a été nettoyée par le liquide de nettoyage, **en ce que** le moyen d'analyse d'image (17) est adapté pour compter les cellules somatiques et/ou gouttelettes de graisse dans l'image de la chambre de mesure (34) nettoyée et **en ce que** le moyen de nettoyage est adapté pour fournir un autre flux d'un liquide de nettoyage à travers la chambre de mesure (34) si des cellules somatiques et/ou gouttelettes de graisse sont détectées dans l'image de la chambre de mesure (34) nettoyée, dans lequel un tel autre processus de nettoyage est démarré automatiquement si le dispositif n'a pas été complètement nettoyé des résidus de lait d'un échantillon de lait précédent.

2. Dispositif selon la revendication 1, **caractérisé en ce que** ledit moyen de nettoyage est adapté pour fournir un flux du liquide de nettoyage à travers le dispositif (14) le long d'un trajet de sorte qu'il vient en contact avec les mêmes composants (15, 30, 33, 34) et les mêmes surfaces que ceux avec lesquels le lait vient en contact dans le dispositif (14),

3. Dispositif selon la revendication 1 ou 2, **caractérisé en ce que** le liquide de nettoyage est adapté pour fournir un flux en contact avec un entonnoir de distribution (15) qui est adapté en premier lieu pour recueillir du lait à tester.

4. Dispositif selon l'une quelconque des revendications précédentes, **caractérisé en ce que** le dispositif (14) comprend un moyen pour fournir une solution de coloration à l'échantillon de lait afin de faciliter le comptage de cellules somatiques ou gouttelettes de graisse dans les images.

5. Dispositif selon l'une quelconque des revendications précédentes, **caractérisé en ce que** le dispositif constitue un composant dans un robot de traite (21).

6. Procédé d'échantillonnage de lait, dans lequel le procédé comprend les étapes consistant à recevoir des échantillons de lait d'animaux dans une chambre de mesure (34), enregistrer des images d'échantillons de lait dans la chambre de mesure (34), et compter des cellules somatiques et/ou gouttelettes de graisse dans les images, fournir un flux d'un liquide de nettoyage à travers la chambre de mesure (34) après qu'un échantillon de lait a été pris, enregistrer au moins une image de la chambre de mesure (34) après qu'elle a été nettoyée par le liquide de nettoyage, compter les cellules somatiques et/ou gouttelettes de graisse dans l'image de la chambre de mesure (34) nettoyée et fournir un autre flux d'un liquide de nettoyage à travers la chambre de mesure (34) si des cellules somatiques et/ou gouttelettes de graisse sont détectées dans la chambre de mesure (34) nettoyée.

7. Procédé selon la revendication 6, **caractérisé par** l'étape consistant à fournir un flux du liquide de nettoyage à travers le dispositif (14) le long d'un trajet de sorte qu'il vient en contact avec les mêmes composants (15, 30, 33, 34) et les mêmes surfaces que ceux avec lesquels le lait vient en contact dans le dispositif (14).

8. Procédé selon la revendication 6 ou 7, **caractérisé par** l'étape consistant à fournir un flux du liquide de nettoyage en contact avec un entonnoir de distribution (15) qui est adapté en premier lieu pour recueillir du lait à tester.

9. Procédé selon l'une quelconque des revendications précédentes 6 à 8, **caractérisé par** l'étape consistant à transporter le liquide de nettoyage au moyen d'une pompe (30) qui est utilisée en premier lieu pour transporter le lait vers la chambre de mesure (34).

10. Procédé selon l'une quelconque des revendications précédentes 6 à 9, **caractérisé par** l'étape consistant à guider le liquide de nettoyage vers la chambre de mesure (34) dans un conduit (33) qui est utilisé en premier lieu pour guider le lait vers la chambre de mesure (34).

11. Procédé selon l'une quelconque des revendications précédentes 6 à 10, **caractérisé par** l'étape consistant à utiliser de l'eau en tant que liquide de nettoyage.

12. Procédé selon l'une quelconque des revendications précédentes 6 à 11, **caractérisé par** l'étape consistant à révéler le résultat du comptage des cellules somatiques et/ou gouttelettes de graisse dans l'image de la chambre de mesure (34) nettoyée sur un écran (36).

13. Procédé selon l'une quelconque des revendications précédentes 6 à 13, **caractérisé par** l'étape consistant à fournir une solution de coloration à l'échantillon de lait afin de faciliter le comptage des cellules somatiques ou gouttelettes de graisse dans les images.
